# EUROPEAN PATENT APPLICATION

(11) **EP 4 692 356 A1**
(43) Date of publication of application: **11.02.2026**
(21) Application number: 24779914.1
(22) Date of filing: 22.03.2024
(51) Int. Cl.: C12N 15/63, C12N 1/21

(54) **PROMOTER SUITABLE FOR DRIVING GENE EXPRESSION IN HYDROGEN-OXIDIZING BACTERIA**

(30) Priority: 24.03.2023 JP 2023047829
(71) Applicant: Logomix, Inc., Tokyo 113-0023 (JP)
(72) Inventor: AIZAWA Yasunori, Tokyo 104-0053 (JP); KUDO Kai, Tokyo 104-0053 (JP)
(74) Representative: Klöckner, Christoph
(86) International application number: PCT/JP2024/011189
(87) International publication number: WO 2024/203795

(57) **Abstract**

The present invention provides a promoter suitable for driving gene expression in a hydrogen-oxidizing bacterium. The present invention also provides a promoter that is suitable for driving gene expression in a hydrogen-oxidizing bacterium and can not drive gene expression in *Escherichia coli.*

## Description

### TECHNICAL FIELD

The present disclosure relates to promoters suitable for driving gene expression in hydrogen-oxidizing bacteria. The present disclosure also relates to promoters that are suitable for driving gene expression in hydrogen-oxidizing bacteria and can substantially not drive gene expression in *Escherichia coli.*

### BACKGROUND ART

Hydrogen-oxidizing bacteria can assimilate carbon dioxide using energy from oxidizing hydrogen, and are expected to be bacteria that enable environmentally friendly and sustainable industrial production. *Ralstonia eutropha* is known as a hydrogen-oxidizing bacterium and is useful for producing the biodegradable bioplastic, polyhydroxybutyrate (PHB) (Non-Patent Literature 1). *Ralstonia eutropha* can also be used for the mass expression of proteins that tend to form inclusion bodies in *Escherichia coli* (Non-Patent Literature 2).

### PRIOR ART DOCUMENTS

### [NON-PATENT LITERATURE]

[Non-Patent Literature 1] Ryu et al., 1997, Biotechnol. Bioeng. 55, 28-32
[Non-Patent Literature 2] Sriram Srinivasan et al., 2002, Appl. Environ. Microbiol., 68, 5925-32

### SUMMARY OF THE INVENTION

The present disclosure provides promoters suitable for driving gene expression in hydrogen-oxidizing bacteria. The present disclosure also provides promoters that are suitable for driving gene expression in hydrogen-oxidizing bacteria and can not drive gene expression in *Escherichia coli.*

The present disclosure provides, for example, the following inventions.
(1) An isolated nucleic acid or vector comprising a promoter of any one of the hydrogen-oxidizing bacterial genes shown in Table 1.
(2) The isolated nucleic acid or vector according to (1), wherein the gene is any gene selected from the group consisting of genes B, F, G, H, I, J, K, L, M, and O in Table 1.
(3) A gene expression vector according to (1) or (2), wherein a gene of interest is operably linked to the promoter.
(4) A composition, comprising the isolated nucleic acid or vector according to any one of (1) to (3), for use in expressing a gene of interest in a hydrogen-oxidizing bacterium.
(5) A composition, comprising the vector according to any one of (1) to (3), for use in cloning in *Escherichia coli* and then expressing a gene of interest in a hydrogen-oxidizing bacterium.
(6) A method for obtaining a hydrogen-oxidizing bacterium, comprising:
   preparing an *Escherichia coli* comprising a vector, wherein the vector comprises a gene of interest operably linked to a promoter of any one of the hydrogen-oxidizing bacterial genes shown in Table 1;
   culturing the *Escherichia coli* comprising the vector to increase the amount of the vector; and
   introducing the obtained vector into a hydrogen-oxidizing bacterium.
(7) A method for expressing a gene of interest in a hydrogen-oxidizing bacterium, comprising:
   preparing a hydrogen-oxidizing bacterium comprising a gene of interest operably linked to a promoter of any one of the hydrogen-oxidizing bacterial genes shown in Table 1; and
   culturing the hydrogen-oxidizing bacterium under conditions suitable for culturing to express the gene of interest in the hydrogen-oxidizing bacterium.
(8) A method for expressing a gene of interest in a hydrogen-oxidizing bacterium, comprising:
   preparing an *Escherichia coli* comprising a vector, wherein the vector comprises a gene of interest operably linked to a promoter of any one of the hydrogen-oxidizing bacterial genes shown in Table 1;
   culturing the *Escherichia coli* comprising the vector to increase the amount of the gene expression vector;
   introducing the obtained vector into a hydrogen-oxidizing bacterium; and
   culturing the obtained hydrogen-oxidizing bacterium under conditions suitable for culturing to express the gene of interest in the hydrogen-oxidizing bacterium.
(9) A composition, comprising a nucleic acid that comprises a promoter of any one of the hydrogen-oxidizing bacterial genes shown in Table 1, for use in the method according to any one of (6) to (8).
(10) The composition according to (9), wherein the nucleic acid further comprises a gene of interest operably linked to the promoter.
(11) A genetically modified hydrogen-oxidizing bacterium, comprising:
   a promoter of any one of the hydrogen-oxidizing bacterial genes shown in Table 1; and
   a gene of interest operably linked to the promoter.
(21) The vector according to any one of (1) to (3), wherein the vector has an origin of replication that can replicate within *Escherichia coli.*
(22) The composition according to (4) or (5), wherein the vector has an origin of replication that can replicate within *Escherichia coli.*
(23) The method according to (6) or (8), wherein the vector has an origin of replication that can replicate within *Escherichia coli.*
(24) The vector according to any one of (1) to (3), wherein the vector has a sequence that can recombine with the genomic DNA of a hydrogen-oxidizing bacterium.
(25) The composition according to (4) or (5), wherein the vector has a sequence that can recombine with the genomic DNA of a hydrogen-oxidizing bacterium.
(26) The method according to (6) or (8), wherein the vector has a sequence that can recombine with the genomic DNA of a hydrogen-oxidizing bacterium.
(27) The vector according to (21), wherein the vector has a sequence that can recombine with the genomic DNA of a hydrogen-oxidizing bacterium.
(28) The composition according to (22), wherein the vector has a sequence that can recombine with the genomic DNA of a hydrogen-oxidizing bacterium.
(29) The method according to (23), wherein the vector has a sequence that can recombine with the genomic DNA of a hydrogen-oxidizing bacterium.
(30) The vector according to any one of (1) to (3), wherein the vector is a gene expression vector.
(31) The composition according to (4) or (5), wherein the vector is a gene expression vector.
(32) The method according to (6) or (8), wherein the vector is a gene expression vector.
(41) A composition, comprising a nucleic acid that comprises a promoter of any one of the hydrogen-oxidizing bacterial genes shown in Table 1, for use in the method according to any one of (23), (26), (29), and (32).
(42) The composition according to (41), wherein the nucleic acid further comprises a gene of interest operably linked to the promoter.
(51) The vector according to any one of (1) to (3).
(52) A composition comprising the vector according to (51).
(53) A composition, comprising the vector according to (51), for use in expressing a gene of interest in a hydrogen-oxidizing bacterium.
(54) A composition, comprising the vector according to (51), for use in cloning in *Escherichia coli* and then expressing a gene of interest in a hydrogen-oxidizing bacterium.

### BRIEF DESCRIPTION OF DRAWINGS

[FIG. 1] FIG. 1 shows an outline of a reporter assay for the identification of control sequences.
[FIG. 2] FIG. 2 shows the structure of the reporter plasmid used in the reporter assay.
[FIG. 3] FIG. 3 shows the results of reporter expression in *Escherichia coli,* or hydrogen-oxidizing bacteria.

### DESCRIPTION OF THE INVENTION

In this specification, a "hydrogen-oxidizing bacterium" is a bacterium that has an ability to oxidize hydrogen and performs carbon assimilation using the energy generated by the oxidation reaction (also called a " Hydrogenotroph"). Hydrogen-oxidizing bacteria (for example, aerobic hydrogen-oxidizing bacteria, Knallgas bacteria) can use hydrogen as an electron donor and oxygen as an electron acceptor, and have an ability to reductively assimilate carbon dioxide to synthesize organic matter. Hydrogen-oxidizing bacteria include, for example, but not limited to, bacteria of the genus Caminibacter, bacteria of the genus Aquifex, bacteria of the genus Ralstonia, and bacteria of the genus Paracoccus. Examples of hydrogen-oxidizing bacteria also include bacteria of the genus Alcaligenes, bacteria of the genus Pseudomonas, bacteria of the genus Bacillus, and bacteria of the genus Hydrogenobacter. Hydrogen-oxidizing bacteria can be grown well in the presence of sufficient nutrients, hydrogen, oxygen, and carbon dioxide. Since hydrogen-oxidizing bacteria perform substance production such as proteins from carbon dioxide, they have the effect of reducing carbon dioxide, and at the same time, since they can produce organic matter such as proteins (for example, animal protein), foods, plastics, chemicals, and biofuels, their utilization is expected. Hydrogen-oxidizing bacteria preferably have a group 1h/5 hydrogenase. In a preferred embodiment, the hydrogen-oxidizing bacterium may be *Ralstonia eutropha.*

In this specification, a "control sequence" refers to a sequence that promotes the transcription of DNA into RNA (particularly mRNA). Examples of control sequences include promoters. In this specification, a "promoter" refers to a part that exists near (particularly upstream of) a DNA region to be expressed and has the function of promoting the transcription of the DNA into RNA (particularly mRNA).

In this specification, a "gene of interest" means a gene that is desired to be expressed in a hydrogen-oxidizing bacterium. In this specification, a gene of interest may be simply referred to as a gene. A gene is a nucleic acid (particularly DNA) that codes for a transcript. The transcript may be, for example, mRNA. The gene of interest may be a gene naturally present in a hydrogen-oxidizing bacterium, or a mutant or derivative of a gene naturally present in a hydrogen-oxidizing bacterium; a gene naturally present in a non-hydrogen-oxidizing bacterial cell, or a mutant or derivative of a gene naturally present in a non-hydrogen-oxidizing bacterial cell; or a gene with an artificial sequence. The gene of interest may be an endogenous gene or an exogenous gene. The gene of interest may be codon-optimized for expression in a hydrogen-oxidizing bacterium. An "endogenous gene" refers to a gene originally present in the genome. An "exogenous gene" refers to a gene introduced into a cell from outside. An exogenous gene may have the same sequence as an endogenous gene or may have a sequence that differs by one or more nucleotides.

In this specification, the term "expression vector" refers to a vector that comprises a polynucleotide of interest and is equipped with a system that enables the polynucleotide of interest to be expressed in a cell into which the vector has been introduced. An expression vector, for example, expresses a gene of interest within the cell of a hydrogen-oxidizing bacterium. An expression vector may comprise an origin of replication necessary for proliferation in a host cell, a drug resistance marker gene for drug selection, a control sequence, and a gene of interest operably linked to the control sequence. An empty expression vector has an origin of replication, a drug resistance marker gene, a control sequence, and a cloning site (for example, a multiple cloning site) where a gene of interest can be operably linked to the control sequence. A cloning site has, for example, one or more restriction enzyme cleavage sites. A fragment can be introduced into the cloning site by mixing it with a fragment having an end suitable for ligation and ligating them. Examples of ends suitable for ligation include blunt ends for blunt ends, and sticky ends with a sequence complementary to the single-stranded portion for sticky ends. In particular, ends obtained by cleavage with the same restriction enzyme can be preferably ligated. A multiple cloning site has cleavage sites for a plurality of restriction enzymes. A multiple cloning site is suitable for integrating fragments that have been cleaved by various restriction enzymes.

In this specification, "operably linked" means linking a gene (DNA) to a control sequence (for example, a promoter) so that its expression is driven.

According to the present disclosure, a control sequence (particularly a promoter) that expresses a gene of interest in a hydrogen-oxidizing bacterium is provided. According to the present disclosure, a control sequence (particularly a promoter) that expresses a gene of interest in a hydrogen-oxidizing bacterium and is transcriptionally inactive in *Escherichia coli* is provided. A control sequence (particularly a promoter) that expresses a gene of interest in a hydrogen-oxidizing bacterium and is transcriptionally inactive in *Escherichia coli* is suitable for being operably linked to a transcript that has a negative effect on *Escherichia coli,* since the gene operably linked to the sequence does not express or expresses only to a negligible extent in *Escherichia coli.*

According to the present disclosure, a nucleic acid comprising a gene of interest operably linked to a control sequence of any one of the hydrogen-oxidizing bacterial genes shown in Table 1 is provided. The nucleic acid may be a nucleic acid that does not occur naturally (or a nucleic acid having a non-natural sequence). According to the present disclosure, a composition comprising the nucleic acid is provided. The composition comprising the nucleic acid preferably has a composition suitable for the preservation and/or stabilization of the nucleic acid.

According to the present disclosure, a vector is provided that comprises a control sequence of any one of the hydrogen-oxidizing bacterial genes shown in Table 1. The vector may have a cloning site for introducing a gene of interest so that it is operably linked to the control sequence. The vector may comprise a gene of interest operably linked to the control sequence. The gene of interest may be, for example, a gene to be introduced into a hydrogen-oxidizing bacterium (preferably, a gene to be expressed in a hydrogen-oxidizing bacterium, and more preferably, a gene that provides some benefit by being introduced and expressed in a hydrogen-oxidizing bacterium). According to the present disclosure, a composition comprising the vector may be provided. The composition has a composition suitable for the preservation and/or stabilization of the vector. In a preferred embodiment, the vector is a gene expression vector. In a preferred embodiment, the vector is a cloning vector.

**[Table 1]**

| Table 1: List of Genes | | |
|---|---|---|
| | Locus tag | Annotation |
| A | E6A55_RS03975 | ssrA: Transfer-messenger RNA |
| B | E6A55_RS17375 | Porin |
| C | E6A55_RS02430 | rnpB: RNA subunit of RNase P |
| D | E6A55_RS04950 | Hypothetical protein |
| E | E6A55_RS16200 | Histone H1-like NDA-binding protein |
| F | E6A55_RS25535 | HU family DNA-binding protein |
| G | E6A55_RS25475 | HU family DNA-binding protein |
| H | E6A55_RS20025 | Flp family Type IVb pilin |
| I | E6A55_RS19135 | Cold shock protein |
| J | E6A55_RS03990 | OmpA family protein |
| K | E6A55_RS30335 | Cold shock protein |
| L | E6A55_RS14400 | DUF883 family protein |
| M | E6A55_RS15575 | Cold shock protein |
| N | E6A55_RS20225 | Hypothetical protein |
| O | E6A55_RS06870 | Hypothetical protein |
| P | E6A55_RS30590 | H-NS histone family protein |
| Q | E6A55_RS17055 | Hypothetical protein |
| R | E6A55_RS32350 | TonB-dependent receptor |
| S | E6A55_RS33115 | H-NS histone family protein |

In one embodiment, the control sequence may be a control sequence of a gene listed in any one of A to S of Table 1. In one embodiment, the control sequence may be a promoter of a gene listed in any one of A to S of Table 1. In a preferred embodiment, the control sequence may be a control sequence, preferably a promoter, of any gene selected from the group consisting of B, F, G, H, I, J, K, L, M, and O of Table 1. In a more preferred embodiment, the control sequence may be a control sequence, preferably a promoter, of any gene selected from the group consisting of B, F, G, H, I, J, and M of Table 1. The control sequence is preferably isolated or cloned.

*Escherichia coli* is preferably used for cloning vectors. In vector cloning, a gene of interest is introduced into a cloning site of a vector, and then the vector after the operation is transformed into *Escherichia coli,* where one vector is introduced into one *Escherichia coli.* When the transformed bacteria are seeded on a solid medium (for example, an agar medium, preferably an LB medium containing agar) and form colonies, one colony originates from one *Escherichia coli,* and therefore, the colony contains only one type of vector. By obtaining this colony and recovering the vector, a vector containing multiple copies can be obtained. This operation is called vector cloning. In the process of vector cloning, the proliferation of *Escherichia coli* may be inhibited by the type of gene inserted into the vector within the *Escherichia coli.* Therefore, it may be preferable for the control sequence to have low activity in *Escherichia coli.* By doing so, the effect of the expression of the inserted gene on the proliferation inhibition of *Escherichia coli* can be minimized. Examples of such a control sequence include a control sequence, preferably a promoter, of any gene selected from the group consisting of B, F, G, H, I, J, and M of Table 1. A promoter is located, for example, upstream of a gene. A promoter is located, for example, from 0 to 300 bp upstream of a gene. A promoter comprises, for example, from 0 to 300 bp upstream of a gene. A promoter may have, for example, the sequence of any one of SEQ ID NOs: 1 to 19. Promoter activity can be detected using cells that have a reporter gene (for example, a drug resistance marker gene, a gene coding for a fluorescent protein, etc.) linked downstream of the promoter. A person skilled in the art can obtain the promoter based on the description in this specification.

In one embodiment, the gene of interest may be a gene of a homospecific or heterospecific hydrogen-oxidizing bacterium. In one embodiment, the gene of interest may be a gene of a non-hydrogen-oxidizing bacterium (for example, a gene of a bacterium, fungus, alga, etc. that is a non-hydrogen-oxidizing bacterium). In one embodiment, the gene of interest may be a hydrogenase, a carbon fixation-related enzyme such as RubisCO, or a synthetic pathway enzyme for producing a specific metabolite. When the expression of the gene of interest has a negative effect on the survival or proliferation of *Escherichia coli,* it is preferable to use a control sequence that substantially does not drive the expression of the gene of interest in *Escherichia coli.* The gene of interest is isolated.

According to the present disclosure, an *Escherichia coli* comprising the nucleic acid or vector of the present disclosure is provided. According to the present disclosure, a hydrogen-oxidizing bacterium comprising the nucleic acid or vector of the present disclosure is provided. According to the present disclosure, a composition comprising the *Escherichia coli* of the present disclosure (for example, a culture, and a glycerol stock) is included. According to the present disclosure, a composition comprising the hydrogen-oxidizing bacterium of the present disclosure (for example, a culture, and a glycerol stock) is included. In one embodiment, the *Escherichia coli* may be JM109, DH5α^{™}, DH10B^{™}, BL21, XL-1Blue, XL-10Gold, SURE^{®}, Mach1^{™}-T1^{®}, NEB^{®} Turbo, and ABLE^{®} C/K.

*Escherichia coli* can be cultured in or on a medium suitable for culturing (for example, LB medium). The medium may be a liquid medium or a solid medium (for example, a solid medium containing agar). Transformation of *Escherichia coli* can be achieved by known conventional techniques. It can be performed by contacting a nucleic acid or a vector with competent *Escherichia coli* cells (which can be obtained by, for example, calcium chloride treatment), giving a heat shock (for example, within about 1 minute at about 42°C), standing on ice, and then adding a culture medium. A vector to be cloned in *Escherichia coli* typically has an antibiotic (for example, ampicillin) resistance gene, and by culturing in the presence of an antibiotic (for example, ampicillin), *Escherichia coli* transformed with the vector *(i.e., Escherichia coli* comprising the vector) can be obtained. Cloning can be performed by seeding the obtained *Escherichia coli* on an agar medium to form colonies.

Hydrogen-oxidizing bacteria can be cultured in or on a medium suitable for culturing (for example, NR medium, and fructose-added MS medium). The medium may be a liquid medium or a solid medium (for example, a solid medium containing agar). An NR medium may comprise, for example, protein hydrolysates such as tryptone and peptone, and extracts (for example, meat extract, fish extract (for example, bonito extract), yeast extract, and yeast extract). The NR medium preferably contains tryptone, yeast extract, and bonito extract. The NR medium may have, for example, the composition shown in Table 3. The fructose-added MS medium may have, for example, the composition of Table 4. A person skilled in the art can design or select and obtain a medium suitable for culturing hydrogen-oxidizing bacteria.

Gene introduction into a hydrogen-oxidizing bacterium can be carried out, for example, by mixing and culturing the hydrogen-oxidizing bacterium with *Escherichia coli* having a plasmid. By doing so, the plasmid in the *Escherichia coli* is transferred to the hydrogen-oxidizing bacterium by conjugation. This type of transformation method is called the conjugation method. In this way, the single type of plasmid can be introduced into the hydrogen-oxidizing bacterium simply by mixing and culturing the hydrogen-oxidizing bacterium with *Escherichia coli* derived from a single *Escherichia coli* colony having a single type of plasmid. The conjugation method is highly convenient because the plasmid in *Escherichia coli* can be introduced into the hydrogen-oxidizing bacterium without the need to purify the plasmid DNA from the *Escherichia coli* colony.

According to the present disclosure, a method for obtaining a hydrogen-oxidizing bacterium is provided, the method comprising:
preparing an *Escherichia coli* comprising a vector, wherein the vector comprises a gene of interest operably linked to a promoter of any one of the hydrogen-oxidizing bacterial genes shown in Table 1;
culturing the *Escherichia coli* comprising the vector to increase the amount (total amount) of the vector; and
introducing the obtained vector into a hydrogen-oxidizing bacterium.

The *Escherichia coli* comprising a vector can be obtained as described in this specification or by known conventional techniques. The culturing of *Escherichia coli* can be carried out as described in this specification or by known conventional techniques. The introduction of the obtained vector into a hydrogen-oxidizing bacterium can be carried out, for example, by mixing and culturing the cultured *Escherichia coli* with the hydrogen-oxidizing bacterium. The vector may be a gene expression vector, a vector for recombination use, or a cloning vector. It is preferable that the gene expression vector has an origin of replication that can replicate within the hydrogen-oxidizing bacterium. The gene expression vector may not have a sequence that can recombine with the genomic DNA of the hydrogen-oxidizing bacterium. It is preferable that the vector for recombination use has a sequence that can recombine with the genomic DNA of the hydrogen-oxidizing bacterium. The vector for recombination use may have an origin of replication that can replicate within the hydrogen-oxidizing bacterium, but it may not.

Examples of a sequence that can recombine with the genomic DNA of a hydrogen-oxidizing bacterium include (i) a sequence that has an upstream homology arm and a downstream homology arm that can each undergo homologous recombination with a region adjacent to the upstream and downstream target regions, respectively, of the hydrogen-oxidizing bacterium, and that comprises a gene of interest operably linked to a promoter of any one of the hydrogen-oxidizing bacterial genes shown in Table 1 between the upstream homology arm and the downstream homology arm, and that, when introduced into a hydrogen-oxidizing bacterium, can insert the gene of interest operably linked to a promoter of any one of the hydrogen-oxidizing bacterial genes shown in Table 1 into the genome of the hydrogen-oxidizing bacterium by homologous recombination with the upstream and downstream target regions, or (ii) a sequence that has two sequences that can each undergo recombination with the target sequences of a site-specific recombinase placed upstream and downstream, respectively, of a target region of the hydrogen-oxidizing bacterium, and that comprises a gene of interest operably linked to a promoter of any one of the hydrogen-oxidizing bacterial genes shown in Table 1 between the two sequences, and that can undergo recombination with the target region of the hydrogen-oxidizing bacterium to insert the gene of interest operably linked to the promoter into the target region.

According to the present disclosure, a hydrogen-oxidizing bacterium comprising a gene of interest operably linked to a promoter of any one of the hydrogen-oxidizing bacterial genes shown in Table 1 and a method for culturing the hydrogen-oxidizing bacterium are provided. Culturing can be performed under conditions suitable for culturing hydrogen-oxidizing bacteria (preferably, under conditions where the hydrogen-oxidizing bacterium can assimilate carbon dioxide). Conditions suitable for culturing hydrogen-oxidizing bacteria include nutrient conditions such as sufficient nutrients, hydrogen, oxygen (for example, 21% or less, 20% or less, 15% or less, 10% or less, or 5% or less, preferably about 2% to about 5%, for example, about 4%), and carbon dioxide, as well as temperature, humidity, and atmospheric pressure conditions. The concentration of the mixed gas used for culturing hydrogen-oxidizing bacteria can be set to hydrogen concentration: oxygen concentration: carbon dioxide concentration = about 8: about 1: about 1. It is also common to keep a part of the mixed gas dissolved in the medium (by stirring, etc., under gas contact) so that it can be used by the hydrogen-oxidizing bacteria. In one embodiment, the gene of interest operably linked to the promoter is integrated into the genomic DNA of the hydrogen-oxidizing bacterium. In one embodiment, the gene of interest operably linked to the promoter is integrated into a vector within the hydrogen-oxidizing bacterium.

According to the present disclosure, a method for expressing a gene of interest in a hydrogen-oxidizing bacterium is provided, the method comprising:
preparing a hydrogen-oxidizing bacterium comprising a gene of interest operably linked to a promoter of any one of the hydrogen-oxidizing bacterial genes shown in Table 1; and
culturing the hydrogen-oxidizing bacterium under conditions suitable for culturing (preferably, under conditions where the hydrogen-oxidizing bacterium can assimilate carbon dioxide) to express the gene of interest in the hydrogen-oxidizing bacterium.

According to the present disclosure, the hydrogen-oxidizing bacterium has a genomic DNA comprising a gene of interest operably linked to a promoter of any one of the hydrogen-oxidizing bacterial genes shown in Table 1. In one embodiment, the gene of interest is an endogenous or exogenous gene, and is preferably an exogenous gene. In one embodiment, the gene of interest is a gene naturally present in a hydrogen-oxidizing bacterium, or a mutant or derivative of a gene naturally present in a hydrogen-oxidizing bacterium; a gene naturally present in a non-hydrogen-oxidizing bacterial cell, or a mutant or derivative of a gene naturally present in a non-hydrogen-oxidizing bacterium; or an artificially designed gene. In one embodiment, the gene of interest has a sequence that differs by one or more nucleotides from the endogenous gene. In one embodiment, the hydrogen-oxidizing bacterium comprises two or more, three or more, or four or more promoters of any one of the hydrogen-oxidizing bacterial genes shown in Table 1.

According to the present disclosure, a nucleic acid (for example, a vector) is provided. The nucleic acid (for example, a vector) has an upstream homology arm and a downstream homology arm that can each undergo homologous recombination with a region adjacent to the upstream and downstream target regions, respectively, of the hydrogen-oxidizing bacterium, and that comprises a gene of interest operably linked to a promoter of any one of the hydrogen-oxidizing bacterial genes shown in Table 1 between the upstream homology arm and the downstream homology arm, and that, when introduced into a hydrogen-oxidizing bacterium, can insert the gene of interest operably linked to a promoter of any one of the hydrogen-oxidizing bacterial genes shown in Table 1 into the genome of the hydrogen-oxidizing bacterium by homologous recombination with the upstream and downstream target regions.

According to the present disclosure, a method for modifying a hydrogen-oxidizing bacterium is provided, comprising:
introducing, into a hydrogen-oxidizing bacterium, a nucleic acid (for example, a vector) comprising an upstream homology arm and a downstream homology arm, each capable of homologous recombination with a region adjacent to upstream and downstream of a target region of a hydrogen bacterium, and comprising, between said upstream homology arm and downstream homology arm, a gene of interest operably linked to any one of the promoters of hydrogen bacterial genes shown in Table 1, wherein, when introduced into the hydrogen bacterium, the gene of interest operably linked to any one of the promoters of hydrogen bacterial genes shown in Table 1 can be inserted into the genome of the hydrogen bacterium through homologous recombination with the upstream and downstream of the target region to induce homologous recombination within the hydrogen-oxidizing bacterium to insert the gene of interest operably linked to a promoter of any one of the hydrogen-oxidizing bacterial genes shown in Table 1 into the genome of the hydrogen-oxidizing bacterium. In one embodiment, the method for modifying a hydrogen-oxidizing bacterium further comprises obtaining the nucleic acid (for example, a vector). Obtaining the nucleic acid (for example, a vector) may include, for example, introducing the nucleic acid (for example, a vector) into *Escherichia coli* and cloning or amplifying the nucleic acid (for example, a vector) within the *Escherichia coli.* In one embodiment, the nucleic acid (for example, a vector) has an origin of replication that can replicate within *Escherichia coli.*

According to the present disclosure, a nucleic acid (for example, a vector) is provided. The nucleic acid (for example, a vector) has two sequences that can each undergo recombination with the target sequences of a site-specific recombinase placed upstream and downstream, respectively, of a target region of the hydrogen-oxidizing bacterium, and that comprises a gene of interest operably linked to a promoter of any one of the hydrogen-oxidizing bacterial genes shown in Table 1 between the two sequences, and that can undergo recombination with the target region of the hydrogen-oxidizing bacterium to insert the gene of interest operably linked to the promoter into the target region. In one embodiment, the hydrogen-oxidizing bacterium may have a site-specific recombinase and/or a nucleic acid that codes for a site-specific recombinase in order to cause recombination.

According to the present disclosure, a method for modifying a hydrogen-oxidizing bacterium is provided, comprising:
introducing, into a hydrogen-oxidizing bacterium, a nucleic acid (for example, a vector), wherein the nucleic acid (for example, a vector) has two sequences that can each undergo recombination with the target sequences of a site-specific recombinase placed upstream and downstream, respectively, of a target region of the hydrogen-oxidizing bacterium, and that comprises a gene of interest operably linked to a promoter of any one of the hydrogen-oxidizing bacterial genes shown in Table 1 between the two sequences, and that can undergo recombination with the target region of the hydrogen-oxidizing bacterium to insert the gene of interest operably linked to the promoter into the target region to induce recombination by a site-specific recombinase within the hydrogen-oxidizing bacterium to insert the gene of interest operably linked to a promoter of any one of the hydrogen-oxidizing bacterial genes shown in Table 1 into the genome of the hydrogen-oxidizing bacterium. In one embodiment, the method for modifying a hydrogen-oxidizing bacterium further comprises obtaining the nucleic acid (for example, a vector). Obtaining the nucleic acid (for example, a vector) may include, for example, introducing the nucleic acid (for example, a vector) into *Escherichia coli* and cloning or amplifying the nucleic acid (for example, a vector) within the *Escherichia coli.* In one embodiment, the nucleic acid (for example, a vector) has an origin of replication that can replicate within *Escherichia coli.*

According to the present disclosure, a composition, comprising a nucleic acid that comprises a promoter of any one of the hydrogen-oxidizing bacterial genes shown in Table 1, for use in any of the above methods, is provided. The nucleic acid may further comprise a gene of interest operably linked to the promoter. The nucleic acid may be integrated into one or more selected from the group consisting of a cloning vector, a homologous recombination vector, and a gene expression vector.

In any of the above embodiments, it is preferable that the gene of interest operably linked to a promoter of any one of the hydrogen-oxidizing bacterial genes shown in Table 1 may be contained in a gene expression vector. In the above embodiments, the gene of interest operably linked to a promoter of any one of the hydrogen-oxidizing bacterial genes shown in Table 1 may be contained in a gene expression cassette in a gene expression vector. Any of the above embodiments may be carried out *in vitro.*

A composition comprising a nucleic acid has conditions for stably preserving the nucleic acid. A composition comprising a nucleic acid may contain a solvent (especially water). A composition comprising a nucleic acid may further contain a pH buffer. A composition comprising a nucleic acid may contain a chelator (for example, a bivalent metal chelator, especially ethylenediaminetetraacetic acid (EDTA) or glycol-ether-diamine-tetraacetic acid (EGTA)). A composition comprising a nucleic acid may be provided in a frozen state. A composition comprising a nucleic acid may be provided in a dried state (for example, naturally dried or freeze-dried). A composition comprising a nucleic acid may be provided as an aqueous solution.

A composition comprising *Escherichia coli* or hydrogen-oxidizing bacteria may contain a medium. A composition comprising *Escherichia coli* or hydrogen-oxidizing bacteria may contain a dispersion medium (especially water). A composition comprising *Escherichia coli* or hydrogen-oxidizing bacteria may further contain a pH buffer. A composition comprising *Escherichia coli* or hydrogen-oxidizing bacteria may contain a chelator (for example, a bivalent metal chelator, especially ethylenediaminetetraacetic acid (EDTA) or glycol-ether-diamine-tetraacetic acid (EGTA)). A composition comprising *Escherichia coli* or hydrogen-oxidizing bacteria may contain an isotonic agent. A composition comprising *Escherichia coli* or hydrogen-oxidizing bacteria may contain glycerol. A composition comprising *Escherichia coli* or hydrogen-oxidizing bacteria may be provided in a frozen state (for example, as a glycerol stock). A composition comprising *Escherichia coli* or hydrogen-oxidizing bacteria may be provided as an aqueous solution.

### EXAMPLES

### Example 1: Identification of highly expressed genes

Hydrogen-oxidizing bacteria (*Ralstonia eutropha* H16) were cultured in a fructose-added MS medium or a low-hydrogen gas-aerated MS medium, and cells were collected at the log growth phase and the stationary phase, respectively. RNA was extracted from the collected bacterial cells, and RNA-Seq was performed. Nineteen genes that were highly expressed under all of these conditions were identified. The identified genes were as shown in Table 2.

**[Table 2]**

| Table 2: Genes identified based on high expression level | | | | | |
|---|---|---|---|---|---|
| | Locus tag | genome | Location | | Annotation |
| | | | Start | End | |
| A | E6A55_RS03975 | chromosome 1 | 857370 | 857012 | ssrA : transfer-messenger RNA |
| B | E6A55_RS17375 | chromosome 1 | 3680681 | 3679572 | porin |
| C | E6A55_RS02430 | chromosome 1 | 507966 | 508306 | rnpB: RNA subunit of RNase P |
| D | E6A55_RS04950 | chromosome 1 | 1068113 | 1068406 | hypothetical protein |
| E | E6A55_RS16200 | chromosome 1 | 3436415 | 3435843 | histone H1-like DNA-binding protein |
| F | E6A55_RS25535 | chromosome 2 | 1406195 | 1406653 | HU family DNA-binding protein |
| G | E6A55_RS25475 | chromosome 2 | 1395893 | 1396174 | HU family DNA-binding protein |
| H | E6A55_RS20025 | chromosome 2 | 206472 | 206648 | Flp family type IVb pilin |
| I | E6A55_RS19135 | chromosome 2 | 2845 | 2642 | cold-shock protein |
| J | E6A55_R503990 | chromosome 1 | 859009 | 859662 | OmpA family protein |
| K | E6A55_R530335 | chromosome 2 | 2503827 | 2504030 | cold-shock protein |
| L | E6A55_RS14400 | chromosome 1 | 3047362 | 3047637 | DUF883 family protein |
| M | E6A55_RS15575 | chromosome 1 | 3308009 | 3308215 | cold-shock protein |
| N | E6A55_RS20225 | chromosome 2 | 248460 | 248233 | hypothetical protein |
| O | E6A55_RS06870 | chromosome 1 | 1471922 | 1471563 | hypothetical protein |
| P | E6A55_RS30590 | chromosome 2 | 2553277 | 2553585 | H-NS histone family protein |
| Q | E6A55_RS17055 | chromosome 1 | 3613799 | 3614128 | hypothetical protein |
| R | E6A55_RS32350 | plasmid pHG1 | 26,821 | 28839 | TonB-dependent receptor |
| S | E6A55_RS33115 | plasmid pHG1 | 196964 | 197275 | H-NS histone family protein |

### Example 2: Construction of an expression level evaluation system using a reporter plasmid and evaluation of the driving force of gene expression by each promoter

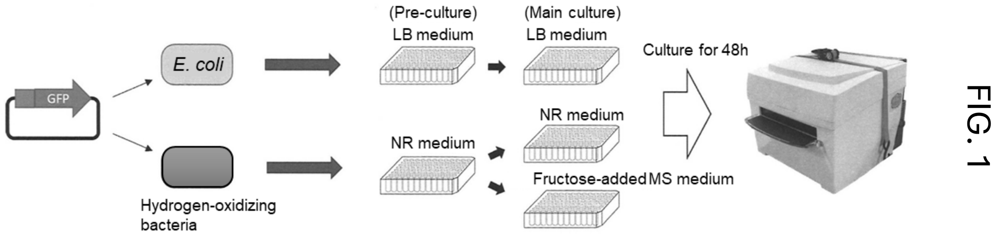

Next, the ability of each promoter to drive gene expression under various conditions was evaluated. Specifically, a GFP reporter plasmid was constructed, introduced into *Escherichia coli* and hydrogen-oxidizing bacteria, respectively, and the expression level was measured based on the GFP fluorescence intensity (see FIG. 1). Pg25 and PphaC1 were used as control promoters (ACS Synth Biol. 2018; 7(8): 1918-28). Pg25 is a promoter derived from phage T5 and has the activity to drive gene expression within *Escherichia coli* and hydrogen-oxidizing bacteria. PphaC1 is an endogenous promoter of a hydrogen-oxidizing bacterium.

### 1. Preparation of plasmids for promoter assay

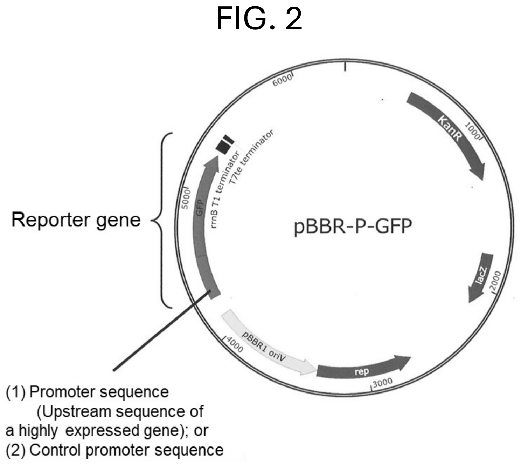

Plasmids for the promoter assay were prepared by integrating a reporter gene cassette, consisting of a DNA fragment of the promoter region of a highly expressed gene (300 bp), a GFP coding sequence DNA fragment, and a terminator sequence DNA, into the shuttle vector pBBR1-MCS_2 (plasmid number 85168) (see FIG. 2).

### 2. Introduction of plasmids for promoter assay

### Escherichia coli (E. coli DH5α)

The introduction of each promoter assay plasmid into *Escherichia coli* was carried out by the following chemical competent cell method.
1. Approximately 100 ng of each promoter assay plasmid was added to *Escherichia coli* chemical competent cells prepared by a general method, and incubated on ice for 30 minutes.
2. 200 µl of LB liquid medium was added, and shake culture was performed for 1 hour at 37°C at 200 rpm.
3. The culture solution was seeded on an LB agar medium (50 µg/mL kanamycin) and incubated at 37°C for about 18 hours to obtain transformants.

### Hydrogen-oxidizing bacterium (Ralstonia eutropha H16)

The introduction of each promoter assay plasmid into the hydrogen-oxidizing bacterium was carried out by the following conjugation method.
1. Various promoter assay plasmids were introduced into an *Escherichia coli* strain containing a conjugation-abilityimparting plasmid by the chemical competent cell method.
2. 500 µl each of the culture solution obtained by seeding the obtained transformants into 2 mL of LB medium (10 µg/mL tetracycline, 50 µg/mL kanamycin) and performing shake culture for 24 hours at 37°C at 200 rpm, and the culture solution obtained by seeding the hydrogen-oxidizing bacterium into 2 mL of LB medium and performing shake culture for 48 hours at 30°C at 200 rpm, were mixed.
3. The mixed culture solution was shake-cultured for 24 hours at 30°C at 200 rpm.
4. The mixed culture solution was seeded on an LB agar medium (10 µg/mL gentamicin, 200 µg/mL kanamycin) and incubated at 30°C for 48-72 hours to obtain transformants.

### 3. Promoter assay method

### Escherichia coli (E. coli DH5α)

1. 500 µL of LB medium (50 µg/mL kanamycin) was dispensed into a 96-deep-well plate, and each promoter assay plasmid transformant was seeded.
2. Shake culture was performed for 24 hours at 37°C at 1400 r/min.
3. 1 mL of LB medium (50 µg/mL kanamycin) was dispensed into a 96-deep-well plate, and 10 µL of the culture solution was seeded.
4. Shake culture was performed for 48 hours at 37°C at 1400 r/min.
5. 100 µL of the culture solution was transferred to a 96-well plate, and OD₆₀₀ and GFP fluorescence were measured with a microplate reader to calculate Relative fluorescence unit (RFU) /OD₆₀₀.

### Hydrogen-oxidizing bacterium (Ralstonia eutropha H16)

1. 500 µL of NR medium (10 µg/mL gentamicin, 200 µg/mL kanamycin) was dispensed into a 96-deep-well plate, and each promoter assay plasmid transformant was seeded.
2. Shake culture was performed for 48 hours at 30°C at 1400 r/min.
3. 1 mL of NR medium (10 µg/mL gentamicin, 200 µg/mL kanamycin) or 1 mL of fructose-added MS medium (10 µg/mL gentamicin, 200 µg/mL kanamycin) was dispensed into a 96-deep-well plate, and 10 µL of the culture solution was seeded into each.
4. Shake culture was performed for 48 hours at 30°C at 1400 r/min.
5. 100 µL of the culture solution was transferred to a 96-well plate, and OD₆₀₀ and GFP fluorescence were measured with a microplate reader to calculate Relative fluorescence unit (RFU) /OD₆₀₀.

### Equipment Used

96-deep-well plate shake culture device
Maximizer M-BR-022UP (Taitec)
Microplate reader
Device Name: EnSpire Multimode Plate Reader (PerkinElmer)
GFP fluorescence measurement conditions: Excitation wavelength 488 nm, Fluorescence wavelength 509 nm

**[Table 3]**

| Table 3: NR Medium Composition | |
|---|---|
| 35% Ehrlich Bonito Extract (Kyokuto Pharmaceutical Industrial Co., Ltd.) | 10 g/L |
| Tryptone (Difco) | 10 g/L |
| Yeast Extract (Difco) | 2 g/L |

**[Table 4]**

| Table 4: Fructose-added MS Medium (MSF Medium) Composition | |
|---|---|
| Na₂HPO₄ · 12H₂O | 9 g/L |
| KH₂PO₄ | 1.5 g/L |
| NH₄Cl | 0.5 g/L |
| MgSO₄ · 7H₂O | 0.2 mg/L |
| CoCl₂ · 6H₂O | 0.218 mg/L |
| FeCl₃ · 6H₂O | 20.5 mg/L |
| CaCl₃ | 7.8 mg/L |
| NiCl₂ · 6H₂O | 0.118 mg/L |
| CrCl₃ · 6H₂O | 0.105 mg/L |
| CuSO₄ · 5H₂O | 0.156 mg/L |
| 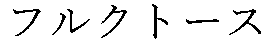 | 10 g/L |

**[Table 5]**

| Table 5: Low-Hydrogen Gas-Aerated MS Medium | |
|---|---|
| Na₂HPO₄ · 12H₂O | 9 g/L |
| KH₂PO₄ | 1.5 g/L |
| NH₄Cl | 0.5 g/L |
| MgSO₄ · 7H₂O | 0.2 mg/L |
| CoCl₂ · 6H₂O | 0.218 mg/L |
| FeCl₃ · 6H₂O | 20.5 mg/L |
| CaCl₃ | 7.8 mg/L |
| NiCl₂ · 6H₂O | 0.118 mg/L |
| CrCl₃ · 6H₂O | 0.105 mg/L |
| CuSO₄ · 5H₂O | 0.156 mg/L |

| | |
|---|---|
| * In addition to the above medium composition, the following mixed gas was supplied to each culture vessel at a flow rate of 15 mL/min for cultivation. The composition of the mixed gas was H₂:O₂:CO₂:N₂ = 4:7:13:76. | |

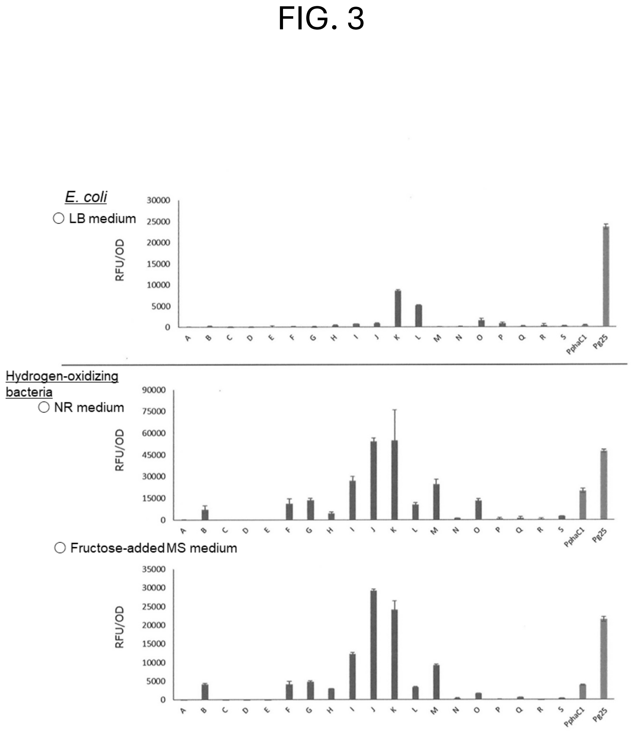

The results were as shown in FIG. 3 and Table 6. As shown in FIG. 3 and Table 6, genes B, F, G, H, I, J, K, L, M, and O had the ability to drive gene expression in a hydrogen-oxidizing bacterium in NR medium and in fructose-containing medium. From these results, it was shown that the promoters of these genes are suitable for gene expression within a hydrogen-oxidizing bacterium.

**[Table 6]**

| Table 6: Gene Expression Levels by Each Promoter | | | | | | | | | |
|---|---|---|---|---|---|---|---|---|---|
| *E. coli* | LB medium | | | Hydrogen-oxidizing bacteria | | NR medium | Hydrogen-oxidizing bacteria | | MSF medium |
| | RFU/OD | SD | | | RFU/OD | SD | | RFU/OD | SD |
| A | 35.54 | 81.65 | | A | -64.55 | 169.24 | A | -175.53 | 16.75 |
| B | 152.92 | 196.88 | | B | 7301.25 | 2543.59 | B | 4195.61 | 202.60 |
| C | -12.96 | 119.58 | | C | -796.20 | 315.98 | C | -309.06 | 72.44 |
| D | -54.40 | 139.06 | | D | -655.48 | 357.18 | D | -359.98 | 126.90 |
| E | 88.22 | 189.03 | | E | -1150.25 | 294.29 | E | -301.49 | 57.93 |
| F | 58.00 | 127.97 | | F | 11322.01 | 3114.96 | F | 4186.94 | 738.12 |
| G | -8.93 | 165.88 | | G | 13557.15 | 1172.32 | G | 4828.06 | 229.39 |
| H | 424.82 | 168.91 | | H | 4349.52 | 1285.93 | H | 2944.89 | 5.96 |
| I | 778.10 | 39.17 | | I | 26996.30 | 2822.68 | I | 12209.86 | 439.75 |
| J | 906.15 | 91.41 | | J | 54018.16 | 2389.36 | J | 29201.94 | 416.56 |
| K | 8579.87 | 242.03 | | K | 54955.54 | 21053.45 | K | 24069.04 | 2344.20 |
| L | 5096.36 | 225.40 | | L | 10368.79 | 1589.87 | L | 3297.39 | 162.67 |
| M | 55.44 | 37.16 | | M | 24680.94 | 3166.79 | M | 9243.26 | 212.64 |
| N | 104.40 | 128.48 | | N | 976.11 | 203.16 | N | 391.44 | 41.70 |
| O | 1565.43 | 460.18 | | O | 13184.95 | 1208.32 | O | 1624.56 | 55.41 |
| P | 861.59 | 242.69 | | P | 732.66 | 735.65 | P | 74.38 | 34.37 |
| Q | 208.46 | 128.80 | | Q | 1187.22 | 898.61 | Q | 589.54 | 64.14 |
| R | 439.06 | 317.09 | | R | 619.92 | 338.71 | R | -216.46 | 118.80 |
| S | 235.89 | 94.11 | | S | 2358.92 | 192.03 | S | 295.01 | 30.89 |
| PphaC1 | 488.15 | 61.58 | | PphaC1 | 19994.23 | 1564.69 | PphaC1 | 3851.87 | 97.94 |
| Pg25 | 23667.42 | 559.77 | | Pg25 | 47408.29 | 1057.97 | Pg25 | 21515.10 | 633.30 |

Next, expression in *Escherichia coli* was focused on. K and L showed expression also in *Escherichia coli,* while other genes showed relatively low expression in *Escherichia coli* (see FIG. 3). Promoters that show low expression in *Escherichia coli* can be used advantageously to prevent a decrease in plasmid yield during plasmid cloning using *Escherichia coli,* for example, by being adopted in a plasmid carrying a protein that, when expressed in *Escherichia coli,* exerts a proliferation inhibitory effect on *Escherichia coli.*

Furthermore, promoters with an RFU/OD of 100 or less in *Escherichia coli* included A, C, D, E, F, G, and M. Among these, F, G, and M had an expression in hydrogen-oxidizing bacteria that was 100 times or more higher than the expression in *Escherichia coli.*

**[Table 7]**

| Table 7: Ratio of Expression in Hydrogen-oxidizing Bacteria to Expression in *Escherichia coli* | | |
|---|---|---|
| | NR/E.coli | MSF/E.coli |
| A | - | - |
| B | 47.75 | 27.44 |
| C | - | - |
| D | - | - |
| E | - | - |
| F | 195.21 | 72.19 |
| G | 1517.58 *¹ | 540.45 *¹ |
| H | 10.24 | 6.93 |
| I | 34.70 | 15.69 |
| J | 59.61 | 32.23 |
| K | 6.41 | 2.81 |
| L | 2.03 | 0.65 |
| M | 445.21 | 166.74 |
| N | 9.35 | 3.75 |
| O | 8.42 | 1.04 |
| P | 0.85 | 0.09 |
| Q | 5.70 | 2.83 |
| R | 1.41 | 0.49 |
| S | 10.00 | 1.25 |
| PphaC1 | 40.96 | 7.89 |
| Pg25 | 2.00 | 0.91 |

| | | |
|---|---|---|
| *1 Absolute value of RFU in hydrogen-oxidizing bacteria / RFU in *Escherichia coli* | | |

### Example of Promoter Sequences (5'→3')

A E6A55_RS03975 
tgccgcggcaagcgccggcgtcgacccgcgccagtgcgtctatgtgggcgatgacctgcgcgacatccaggccggca
aggccgccggcatgatcaccgtcacagccgcctatggctattgcggcgacgccgagccgcccgagacctggggcgcc
gaccacctgattcgccacccggccgagctgatcccgctgctgttgcccgccgtggtcgcctgattgtgtcgcagggg
tatcctggccgttcccggaactccccgcgacagcttgcgtccaaccgggtacaatccagcttcctcact
B E6A55_RS17375 
tttgtcggccgtccgtcacctgaagcgactgcttcaggggaaacgcagcccgactaattcggctgaggtaggccttt
cgtatctgctaatccccgtttttgcaccaaaacggggattttccctcccattcctgttgctcccccacaacaaaaac
cgccagtaagcatggtttacgccgaaaaagccttctgacggccgggggggctggcacataatacgcagcagacgtga
atgtaaatgcgtcgcccaagtacttgggcaaccaagatttcccaatcagttgacaaggaaagtgtcgat
C E6A55_RS02430 
tactcgaaaaactggtgcagccgtacaagtcggtcactgcccatgcatgggtgctgccgctggcgccgcagcaggcg
gcaggcccgtggctggacgtcatgctggcgcaatcccttgccgctgcggcccaggctggcgcgctgcgcggcgggcg
cagttttgcgccgctgccggtgatgggtattccgggctggtgcgacgataacgccgaacctggcttctacgccgata
cctctgtgttccgcccaggacggcgcgccgactgaaggcgccgccaatcggtgttagactgcgggccgc
DE6A55_RS04950 
acgctcaacagcaccccattgctggcaatgtgccggttccgagaatagttcgtataacctccgcccattgtgaagga
ttactttttgttacattcgggggagtccctggtagggttatttcaacgctattaaaaatattgaaattattcctctt
cggccctaaggaaccttttgatagcatgcacgccgctgctgccggataaccgttcgcatcagtgttttcctttacat
atggcttccatatgaggcgcctttcatatggcgcaaccgtttctgtgcattcccatggaaggagtcgtt
E E6A55_RS16200 
gccagctgtccgatatctcctcatttctggccgtgcgccggctcaacgggcggtgcgggctgatcgaatattccagg
gcgcggcacgtgcccgagacccgcttgcgggctcgctccggccgccaagtcaccgccaggggcggtgcgcctgcaac
caaattcattagcgtgcagcagtggtcgggccgcacgccgatgaatcgctcgctcgactggggccgcgctgctgtag
tgcgcctcgggtcggtcgggtcgccaacttaggtcattcgatattcactctctagtgaaggagttatcc
F E6A55_RS25535 
cgtgaccaggcagcaaacggtagcgccgcagcacgcgcatcgctgcgcgcacaggcaccgaagagcctgccgcgaac
gccaaaacgggggtacgtaggccgtattcgcgtttccactgaggcaaagccgcgacaaaacccggggttttcacggg
gttttacgtttcgaatgcacgaaaaccccttgacttacggtactttccgctatgattcagtgggcgtcgagcagacg
attgcgcaacgctgagaagcaatgcgaaacacctgccgacacccatctgttcaacttacgaatatcgat
G E6A55_RS25475 
cgttatggtgcggcgccccatttgtgggaatctatcctatgctgctggtttgccccacgcgcgggccggtctggcgt
tgccgcgctgcgggacggcccggcaaagccggcttctggcaagcgggaaaagcgtggaaaattgcccgctaaccggc
ctcaatcaaggatttgcgggtttttttgtcgcattggtgccactggggattgctctacgcgcgacgggcagattaga
ctgcgccgatcgatggctttgccgcgaggcacgcacatcggtgacgacgaccaacaagggaaatcagcc
H E6A55_RS20025 
attttggctggcattgcggccgtatgaccgcaatgccaggttgcaggccggttcccggccggtctgcctgctctttg
acaacctgtggacccgggcgtgccgggtcgtttcaaacgcgcgtcttaacccctttgggtcattgctatcacccgta
ggaggggtatggttgagacattccatgtcttaaaaagaaagcgcagcaagcgtgaaaagtcgataaaatagcgggcc
acagagcaccgtgataatcgaacgggggaagcgtgatcgcggcaacagatcacttcgggagtggaaatc
I E6A55_RS19135 
ccgttttccaaatttacaaaatatatgttgccaacgcagagagcagaggccgccatttggcagccggaaacgagccg
taccgtaaaattgcttgaaaaacagcccgggatgtgcctaaagtatctatagtgcgcattacgccgcacatgacacc
gcaatccaaagtggttatgatgcgcgctacagtcgagtcaagtcgtgtcaacctcgtcggcttccgttcggtatcta
cgagaactcaacgtgcttgaacggttgtttgcgttgtgtacaagaacttgcttcgaaaaggaaattcct
J E6A55_RS03990 
cttgcgcacacctcccgaggccctcgctgaaacccggaatccctgatgccacaaggctttgcgggcgacacaagacc
tatgttgcacaaacaccacaggcccaaaacccatgcggcgaatcgaatatatttacttcttaggaaacgacccttgt
ggcacaattccccagcgaagagccagacattgttcgaagtggagcatccaccacatcgagaatgttatactccgaag
aatgtatgacttgttttcgtccatttgctcgcagtaaccctgcggtgatctcatcctgagaggagaaat
K E6A55 RS30335 
tgaactgaagctgaagtagaaaacgctaatatctcctatcggaacctctcatcatgattaggcgcttagcctgattg
acacgccgggaaaattcggcagaatgattcccacttgtgcagcgcccatgcggtgtgtcttttggtgttgtcgttcc
gtgacgttttcggttgtcacgacagcgacatatcgaagtcatatggaatgcgctggcggggaacataaaaaggatcc
gctgggtgcgttgcacacggtgtataacgagcgttttcgctcattttgaaattcaaggttgagatagat
L E6A55_RS14400 
tctcctgatttcggggtggaaccagagtatccggattgatatccaacccaaacgaatataaagtcactacaaaatca
ctcgaagtcataaagacattttgtgacattgatcacgcgtgctttccggcatgcttgcgataccaggcactgcagag
tcgcgcttgctggcgtgtagccttgaaaccaccgccgcccgccacatatgctaagtatcagaccgacaaatgccgtt
agcctcggagcgtagaaaatgtcagaatccgcacaaaccgaaccaaccgcccgcaaacaggagaaactg
M E6A55_RS15575 
cctgctggcagtggggaatccgccaacttgacagtatcctgctttgccagaagaatcgttcgcacacctgccgacgg
ccaacgagccaaagcaacagccgagacaggtgatccgtgagcagggaggccacggccgccgcggcggcgagtcagca
tcgagactgtccgccacgcgattgcacctcgccgcgcgtcatcgccggccgcgcctcaggacgcggcaggtcatggc
 gccgcagtccgtgccgtctggccggcaaggcttccccgctttgaaatctgctatttcgtgggggagtat
N E6A55_RS20225 
gttggaagcggatgcggcagacccggaaccgggacgcgccggtagccgcacttcgcggggctggcgctatggtatcg
caatgccggccttgcgcagcacctcagcgatgtcgtcaccggcgtcgccagcagatgccatacattcgttaaatttg
tcattttggaaatttcctggcagacagaattaaaaaacgctgaatggcatttctgcgttggatgcaatacattccat
acattgaaaatccagtgcgagcgcatacccgcccccggccgcaccttgcagcaatcctggagtgtgtcc
0 E6A55_RS06870 
cgcgaggggtcatgaccggctgccacctggcagccggtttttttatggctgccagttgaaaatgtgccatcagtcac
ccacatctgccccagaatcgttgcatttgatccggcgcagtacgcaggcaacgccatcggtattcaatacttgtaga
agaaactttcactttgcggacaagcgctgactcggtacattaaccgggtgttccgccacatgtgcccagccgccacc
ggccaggctcggccccaaaaccggattcgcgcaggcgctgcgcgaccgtcaatcgcgaggataagcaag
P E6A55_RS30590 
aaaatctgctggctcatcaggggctccggtagtcgtgtcagagggggctgcgccgggcgcagacaagcagtctaggc
gctaaaggccgggggcgtgcgtattttcattgtccttaaatgcgggcaaaacgggcgatgcccagcccaataccggc
tgcatcgtgggctgtgcgaccgtcaattcaggccagcaaacaatcagtccgtgcaatctccgttattttttgtaaaa
tttttttaggtgaaccgacaccacacttctagtaccatgcagcggcctatttgcagaaaggggatataa
Q E6A55_RS17055 
cgctgtacaagatcgacatgatcatggacggcgacatggatgagctgctgtcggcactggcttcggaacaccaggct
gatcagcttgctgcacttggcgaagagtcgtaagcaccacaaaatgtcgtgcaaccggccttccgccccgaacaccg
cgtaaagtcgccgatatctttgtatttcttgtaaatgcttgaaacagcactggtaggggtaaggcggctgtctataa
tcgatttcgaccggcaaacggagaatggtgccgggtagtcactcccgagaacatacctaggacgaaatc
R E6A55_RS32350 
aatacgtgctaacccacccgaacgcgaagatcccgcaggtacctgctggttcgcgatctgcgcttcggccaagtact
tctgggcagtcttgaaggaggctcaatgattcagtgtgcattcaagattgtgatgaaagccgtagggaagaattgcg
catcgggtcaaaggccgggttgtcgtgacgctccccaggcgctcgcgccgcattgcggcccgtgcctgcgacggccc
tcgacgctggcgatcggagttgcggtaagctgctgcgccatccctcatggcgcctcgatcgccggcggc
S E6A55_RS33115

aggctggttcttatcagttcaaaccgaggggactttgcacccgaatcacgcgcattgttttacggcgctccctttaa
tgcagttctcgctgcccttaccgcatggagcctcactgtggcgcggcggtggtactcagtcgctgtcatactttctc
gctcagcttttttggtcatgattcggagccgccaagcgaatcattggactgctcattacccagaaggttaaagtttt
tatgccctgaagatcaaattggctctaaactacctatctggatctctcgatcggaaaggaaaccagaag

## Claims

1. An isolated nucleic acid or vector, comprising a promoter of any one of the hydrogen-oxidizing bacterial genes shown in Table 1.

2. The isolated nucleic acid or vector according to Claim 1, wherein the gene is any gene selected from the group consisting of genes B, F, G, H, I, J, K, L, M, and O in Table 1.

3. The isolated nucleic acid or vector according to Claim 1 or 2, wherein a gene of interest is operably linked to the promoter.

4. A composition, comprising the isolated nucleic acid or vector according to any one of Claims 1 to 3, for use in expressing a gene of interest in a hydrogen-oxidizing bacterium.

5. A composition comprising the vector according to any one of Claims 1 to 3, for use in cloning in *Escherichia coli* and then expressing a gene of interest in a hydrogen-oxidizing bacterium.

6. A method for obtaining a hydrogen-oxidizing bacterium, comprising:
preparing an *Escherichia coli* comprising a vector, wherein the vector comprises a gene of interest operably linked to a promoter of any one of the hydrogen-oxidizing bacterial genes shown in Table 1;
culturing the *Escherichia coli* comprising the vector to increase the amount of the vector; and
introducing the obtained vector into a hydrogen-oxidizing bacterium.

7. A method for expressing a gene of interest in a hydrogen-oxidizing bacterium, comprising:
preparing a hydrogen-oxidizing bacterium comprising a gene of interest operably linked to a promoter of any one of the hydrogen-oxidizing bacterial genes shown in Table 1; and
culturing the hydrogen-oxidizing bacterium under conditions suitable for culturing to express the gene of interest in the hydrogen-oxidizing bacterium.

8. A method for expressing a gene of interest in a hydrogen-oxidizing bacterium, comprising:
preparing an *Escherichia coli* comprising a vector, wherein the vector comprises a gene of interest operably linked to a promoter of any one of the hydrogen-oxidizing bacterial genes shown in Table 1;
culturing the *Escherichia coli* comprising the vector to increase the amount of the gene expression vector;
introducing the obtained vector into a hydrogen-oxidizing bacterium; and
culturing the obtained hydrogen-oxidizing bacterium under conditions suitable for culturing to express the gene of interest in the hydrogen-oxidizing bacterium.

9. A composition, comprising a nucleic acid that comprises a promoter of any one of the hydrogen-oxidizing bacterial genes shown in Table 1, for use in the method according to any one of Claims 6 to 8.

10. The composition according to Claim 9, wherein the nucleic acid further comprises a gene of interest operably linked to the promoter.

11. A genetically modified hydrogen-oxidizing bacterium, comprising:
a promoter of any one of the hydrogen-oxidizing bacterial genes shown in Table 1; and
a gene of interest operably linked to the promoter.
